**Europäisches Patentamt**

**European Patent Office**

**Office européen des brevets**

(19)

(11) Publication number: **0 189 329**
A2

(12) **EUROPEAN PATENT APPLICATION**

(21) Application number: **86300490.9**

(22) Date of filing: **24.01.86**

(51) Int. Cl.⁴: **A 61 B 17/22, A 61 B 17/39**

(30) Priority: **25.01.85 US 694746**

(43) Date of publication of application: **30.07.86**
**Bulletin 86/31**

(84) Designated Contracting States: **DE FR GB IT SE**

(71) Applicant: **Fischell, Robert E., 1027 McCeney Avenue, Silver Spring Maryland 20901 (US)**
Applicant: **Fischell, Tim A.,, 1027 McCeney Avenue, Silver Spring Maryland 20901 (US)**

(72) Inventor: **Fischell, Robert E., 1027 McCeney Avenue, Silver Spring Maryland 20901 (US)**
Inventor: **Fischell, Tim A.,, 1027 McCeney Avenue, Silver Spring Maryland 20901 (US)**

(74) Representative: **Beresford, Keith Denis Lewis et al, R.G.C. Jenkins & Co. 12-15, Fetter Lane, London EC4A 1PL (GB)**

(54) **A tunneling catheter system for transluminal arterial angioplasty.**

(57) A tunneling catheter system for percutaneous transluminal angioplasty which includes a catheter for advancement through a patient's arterial system to the vicinity of an arterial stenosis and tunneling means disposed within the catheter for tunneling through the arterial stenosis. Means for centering the catheter and for various methods of tunneling through the catheter are also provided.

## A TUNNELING CATHETER SYSTEM
## FOR TRANSLUMINAL ARTERIAL ANGIOPLASTY

FIELD OF INVENTION

This invention is generally in the field of catheters to accomplish arterial, transluminal angioplasty and more specifically, is a catheter system for tunneling a lumen through an arterial occlusion or near occlusion so that the blood flow through that artery can be significantly increased.

BACKGROUND OF THE INVENTION

Atherosclerotic arterial disease is the leading cause of morbidity and mortality in the United States and most other developed countries. Atherosclerosis is a chronic disease process characterized by lipid deposits and fibrosis of the intima, irregularly distributed in large and medium sized arteries. The disease is progressive and most often becomes clinically manifest in the middle-aged and elderly. When severe, the atherosclerotic plaque causes a reduction of the cross sectional area of the arterial lumen, with or without thrombosis. Resultant ischemic manifestations include: angina pectoris, myocardial infarction, stroke, intermittent claudication, gangrene of the lower extremities and renovascular hypertension.

The current management of atherosclerotic disease includes preventative therapy aimed at minimizing known major risk factors such as hypertension, smoking, hypercholesterolemia and diabetes mellitus.

Coronary artery bypass grafting (CABG), carotid enderterectomy and bypass grafting (autogenous vein or synthetic graft) of the iliac, femoral and renal arteries are all well established surgical methods of palliative therapy. Although these procedures are often effective in relieving ischemia, each of these represents a major surgical operation with significant associated morbidity, mortality and expense. CABG, for example, requires the opening of the chest cavity (thoracotomy) and use of cardiopulmonary bypass, with not uncommon postoperative complications including postpericardotomy syndrome, Non-A Non-B hepatitis, stroke and a mortality of approximately one percent (1%) at most centers.

Percutaneous transluminal angioplasty (PTA) by means of a balloon catheter is a relatively new ("non-surgical") procedure with proven efficacy in the relief of atherosclerotic obstruction of the coronary, renal and peripheral arterial circulations. The technique involves the percutaneous passage (under local anesthesia) of a specialized balloon-tipped catheter through the site of arterial narrowing, and inflation of the balloon to reduce the obstruction. This is always done in conjunction with angiographic visualization of the vessel being treated. When successful, this procedure results in a reduction of the arterial stenosis and a decrease in the transstenotic pressure gradient. The mechanism of action is felt to consist of some combination of plaque compression, intimal splitting and medial/adventitial stretching. Healing of the balloon-damaged plaque may involve fibrosis and retraction of the split intimal elements, with further luminal enlargement in the weeks to months following the procedure.

The safety and efficacy of PTA is a function of the vessel being treated, patient selection, and the expertise of the physician performing the procedure. Primary angiographic success, defined as a 20% or greater reduction of stenosis, is now achieved in approximately 80-90% of

- 3 -                                    0189329

attempts in carefully selected patients at experienced centers. The obvious advantage of PTA, compared to surgical palliative therapy, is that it does not require major surgery or general anesthesia with their associated sequelae.

Despite its proven efficacy in the palliation of obstructive atherosclerotic disease, PTA, as it is currently performed, has several important technical limitations. These limitations are particularly true in the application of PTA to the coronary circulation.

Even in the most skilled hands, dilation of an arterial obstruction is currently not achievable in approximately 20% of attempts. The most common cause of failed PTA is the inability to pass either the guide wire or dilating catheter through the site of a tight or eccentric stenosis. This problem is even more common in attempts to dilate the difficult to access right and circumflex coronary arteries. Although technical advances, such as steerable catheters, have reduced the frequency of unsuccessful attempts, inability to cross a tight, eccentric or fully closed stenosis remains a major limitation of PTA.

Attempts at balloon or guide wire passage in vessels which are tightly stenotic may lead to arterial dissection and/or acute occlusion necessitating emergency vascular surgery. This major complication occurs in 6-8% of attempts at coronary angioplasty.

Inability to dilate an obstruction, even after proper balloon positioning and inflation is a second common mode of PTA failure. This problem is most frequently encountered in older plaques which are densely fibrotic and/or calcified.

Restenosis of the obstructed arterial segment following successful PTA is a major problem with the current technique. This problem is more common following PTA of a coronary obstruction (30-35% at one year) than in the peripheral circulation (10-15% at two years). Pharmacologic attempts to reduce the incidence of restenosis have been largely unsuccessful.

Distal embolization of atherosclerotic plaque following balloon PTA occurs in approximately 5% of patients undergoing PTA of lower extremity or renal arteries. Although these emboli are usually clinically insignificant in these vascular territories, such embolization could be catastrophic in the cerebral circulation. For this reason, balloon PTA is considered to be contraindicated for the treatment of obstructive lesions in the arteries of the aortic arch, such as the carotid artery.

In U.S. Patent 4,207,874 (dated June 17, 1980) D.S.J. Choy describes a means for using a laser beam to tunnel through an arterial occlusion by vaporization of the obstruction. The difficulty with Choy's invention is that there is insufficient means to prevent simultaneous destruction of the arterial wall. For example, the Choy invention shows an intense laser beam directed in the forward direction without significant beam attenuation in that direction. If the artery were to curve and the arterial wall was exposed to the laser beam, the wall could also be vaporized which could be a catastrophic result for the patient. Although the Choy invention describes a means for direct visualization of the obstructed region, it does not describe a centering means to guarantee that the laser beam does not illuminate part of the arterial wall. Furthermore, the Choy invention may completely obstruct a partially obstructed artery thereby cutting off blood flow to distal tissues for a significant time period. The result is ischemia which could cause irreparable damage to heart or brain tissue.

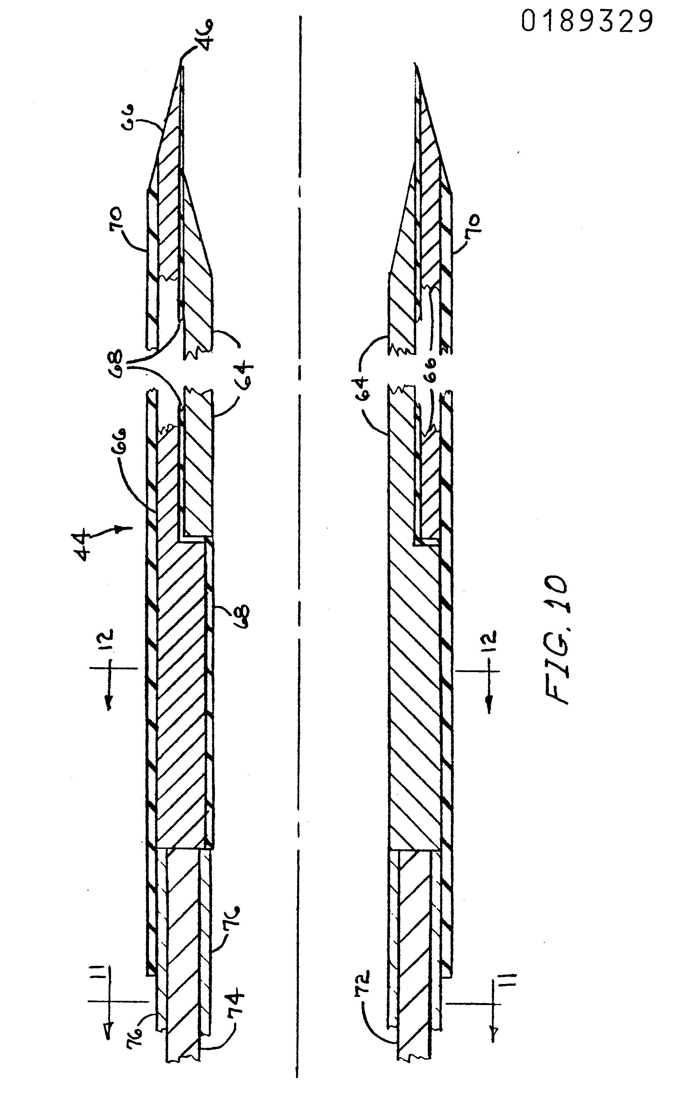

In U.S. Patent 4,273,128 (dated June 16, 1981) B. G. Lary describes a "Coronary Cutting and Dilating Instrument" used for opening a coronary stenosis that is restricting blood flow. The device described by Lary could not be used in a completely occluded artery because its "blunt ovoid tip" could not pass through a completely occluded vessel. Furthermore, the Lary invention does not have any means to prevent its cutting blade from cutting through the arterial wall as well as the occluding stenosic material. Furthermore, there is no means taught in the Lary patent for centering the cutting blade within the arterial walls. Thus, if the probe wire 13 (of FIG. 10) of the Lary invention guides the knife through a highly eccentric lumen within the stenotic plaque, its knife blade would surely cut through the arterial wall which would have serious adverse effects for the patient.

Thus, it is the goal of the present invention to eliminate the numerous shortcomings of the prior art in order to provide a device which can safely tunnel a clean hole through a partially or fully occluded arterial stenosis even if the narrowed stenotic passage is located eccentrically within the arterial wall.

SUMMARY OF THE INVENTION

The tunneling angioplasty catheter system described herein is capable of being placed within an arterial lumen and then advanced until it is in contact with an occlusion. The centering catheter (of the catheter system) is then expanded until its spokes engage the inner arterial wall just proximal to the occlusion. The expanding spokes provide a centering means for a tunneling catheter. With the aid of biplanar, image intensified x-ray fluoroscopy, the tunneling catheter is then advanced into the plaque which forms the arterial occlusion. The tunneling catheter is generally in the shape of a hollow cylinder with a sharp circular cutting edge. A bore cylinder of the plaque is caused to enter an interior volume of the advancing tunneling catheter. The bore cylinder is held in place in that volume by means of a suction applied at the proximal end of the tunneling catheter whose hollow lumen is in fluid communication with the distal end of the catheter. When the tunneling catheter is removed from the body the bore cylinder of plaque is also removed, thus opening the arterial lumen.

Several methods are possible to promote cutting by the sharpened distal end which is the cutting edge of the cylindrical tunneling catheter. One method is merely to advance the extremely sharp distal cutting edge; another method is to rotate the tunneling catheter's cutting edge as it is advanced. Another method is to apply a high energy ultrasonic vibration into the tunneling catheter from its proximal end causing the sound energy to be readily transmitted by conduction into the catheter's cutting edge. Another method for deftly cutting through the plaque is by applying an electrocautery alternating current to the cutting edge. Still another method is by the use of a fiber optic through which is transmitted a high energy laser light beam which is used to vaporize the plaque within the tunneling catheter's distal interior volume.

Once a tunnel has been made in the plaque, a balloon angioplasty catheter could be advanced into the partially opened occlusion. The balloon can then be expanded to further dilate the opening as is well known in the art of percutaneous, transluminal balloon angioplasty. The tunnel that is bored in the plaque may be of sufficient diameter itself to allow adequate blood flow without the subsequent use of an angioplasty balloon catheter.

Thus an object of this invention is to tunnel a hole through an arterial occlusion, particularly an occlusion that is nearly fully or totally occluded or which has its narrowed passageway eccentrically located relative to the center of the arterial lumen.

Another object of this invention is to first use a tunneling catheter system to bore a tunnel into the plaque and then use a balloon angioplasty catheter to further enlarge the lumen of the occluding plaque.

Still another object of the present invention is to employ a sharpened, cylindrical edge of a tunneling catheter to cut through the plaque.

Still another object of the present invention is to utilize ultrasonic vibration of the cylindrical cutting edge to facilitate its ability to cut through the plaque.

Still another object of the present invention is to utilize an electrocautery to apply an electric current to the cutting edge of the tunneling catheter to enhance its ability to cut through the occlusion.

Still another object of the present invention is to utilize a carefully guided fiber optic through which a laser light beam transmitted to vaporize plaque that is within an interior volume at the distal end of the tunneling catheter.

Still another object of the present invention is to apply this technique to any occluded artery including the coronary arteries, the carotid artery, the renal artery and the arteries of the legs and arms.

Other objects, features and advantages of the present invention will be apparent from the following descriptions considered in conjunction with the several drawings which collectively form the specification of this invention.

FIG. 1 illustrates a system of catheters placed through the lumen of the aorta into the left coronary artery.

FIG. 2 illustrates the distal end of the centering catheter in its expanded position.

FIG. 3 shows the cross-section of the centering catheter at a point where the unextended spokes are within the outside tube of the centering catheter.

FIG. 4 shows the cross-section of the distal end of the centering catheter showing how the spokes engage the interior surface of the arterial wall.

FIG. 5 illustrates one embodiment of the tunneling catheter within the inside tube of the centering catheter as it is positioned to tunnel through a nearly fully occluded segment within an artery.

FIG. 6 shows an alternative embodiment of the tunneling catheter specifically showing the design for the distal tip of a tunneling catheter that could utilize an electrocautery current to enhance its ability to cut through plaque.

FIG. 7 shows the cross-section of the connecting wires for the electrocautery tunneling catheter of FIG. 6.

FIG. 8 shows the cross-section near the distal end of the electrocautery tunneling catheter of FIG. 6.

FIG. 9 illustrates the synchronous application of electrocautery current with the refractory period of the heart.

FIG. 10 illustrates a bipolar electrocautery tunneling catheter.

FIG. 11 shows the cross-section of the connecting wires to the electrocautery catheter of FIG. 10.

FIG. 12 shows the cross-section near the distal end of the electrocautery tunneling catheter of FIG. 10 illustrating how the two electrodes are electrically insulated from each other.

FIG. 13 shows another alternative embodiment of the tunneling catheter that would employ a fiber optic to cause a divergent laser beam to be transmitted inside the distal interior volume of the tunneling catheter in order to vaporize the plaque in that region.

FIG. 14 is a cross sectional view of the proximal portion of the catheter system illustrating the means for inserting the centering catheter and expanding its spokes and for inserting and advancing the tunneling catheter.

FIG. 15 is a top view of that portion of the proximal end of the catheter system that is used to adjust the diameter of the spokes of the centering catheter by pulling back on the outside tube of the centering catheter relative to its inside tube.

FIG. 16 is a top view of that portion of the proximal end of the catheter system that is used to set the stroke length for tunneling through the plaque by means of a tunneling catheter.

DETAILED DESCRIPTION OF THE INVENTION

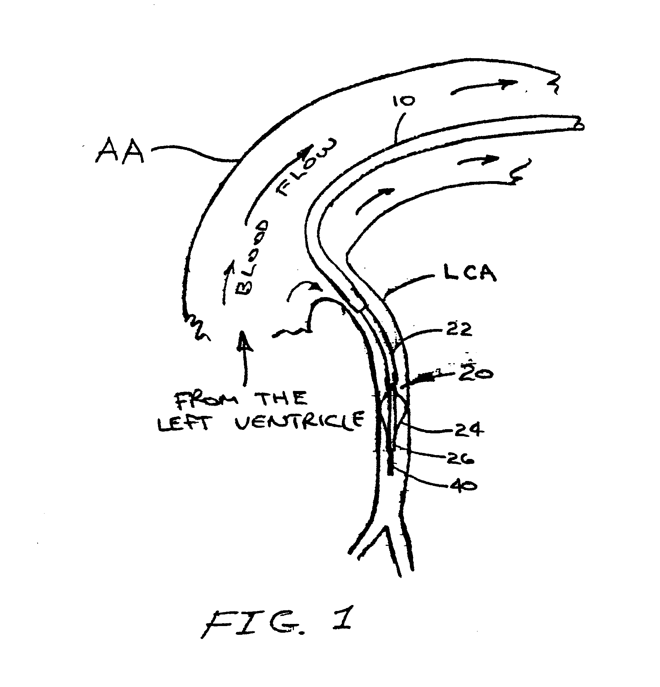

FIG. 1 shows the configuration of several catheters as they pass from the ascending aorta AA into the left coronary artery LCA. To accomplish this, a surgical incision is typically made in the upper portion of the right leg where access to the femoral artery can be accomplished. The guiding catheter 10 is then transluminally advanced through the descending aorta, then into the ascending aorta AA and finally entering the left coronary artery LCA. Of course, it is well known in the medical art to place such a guiding catheter into a large variety of arteries including the heart's posterior descending or circumflex arteries, or the carotid, femoral, renal or any other artery in which a stenotic condition can occur. Thus, although this specification will specifically describe tunneling through a coronary artery, it is also within the teachings of this invention to utilize the techniques described herein for opening of any of the arteries of the body of a human subject or any other living organism that suffers the affliction of arterial occlusions.

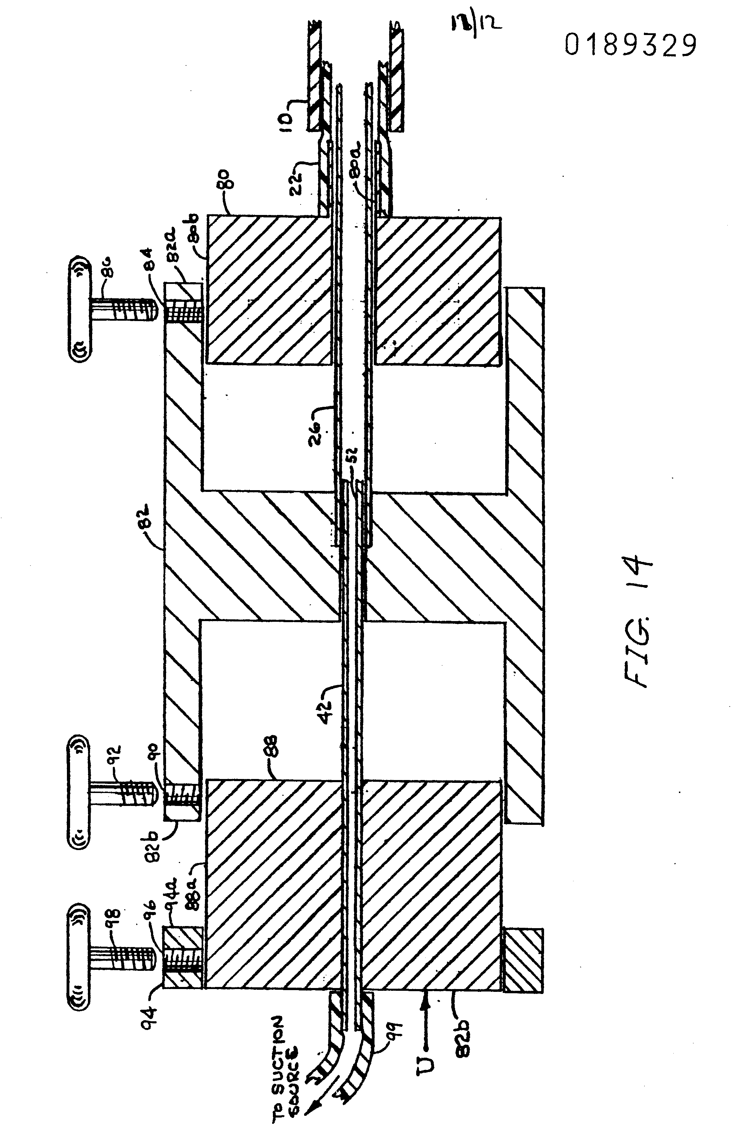

Returning now to FIG. 1, once the guiding catheter 10 has been placed in the left coronary artery LCA, a centering catheter 20 having an outside tube 22 is advanced through the guiding catheter 10 until its distal end is stopped by the occlusion. The spokes 24 of the centering catheter 20 are then extended to engage the artery's interior surface using the proximal portion of the apparatus shown in FIGS. 14, 15, and 16. A radio-opaque substance is then transmitted through the guiding catheter to better define the site of the occlusion. This occlusion would undoubtedly have been found from a prior angiographic study, so that its location would actually be reconfirmed prior to the tunneling activity. A tunneling catheter 40 is then advanced through the inside tube 26 of the centering catheter 20 until the forward motion of the tunneling catheter is also stopped by the occlusion. Using biplanar, image intensified x-ray fluoroscopy, the accurate centering of the tunneling catheter is verified and the length of the occlusion is accurately determined. The proximal portion of this system for tunneling angioplasty (shown in FIGS. 14, 15, and 16) is then used to set the stroke length for the tunnel that is to be bored in the occlusion. The tunneling catheter is then advanced by that length, and a bore cylinder or bore volume is removed. Angiography can then be utilized to determine if the tunnelled lumen is sufficiently large to allow adequate blood flow. If it is, the procedure would be terminated. If not, a balloon angioplasty catheter could be advanced into the tunneled lumen and then expanded to further increase the flow of blood. Since the tunneled lumen prior to insertion of a balloon catheter would be of reasonable size and centrally located within the artery, use of the balloon angioplasty technique at that point would not be difficult.

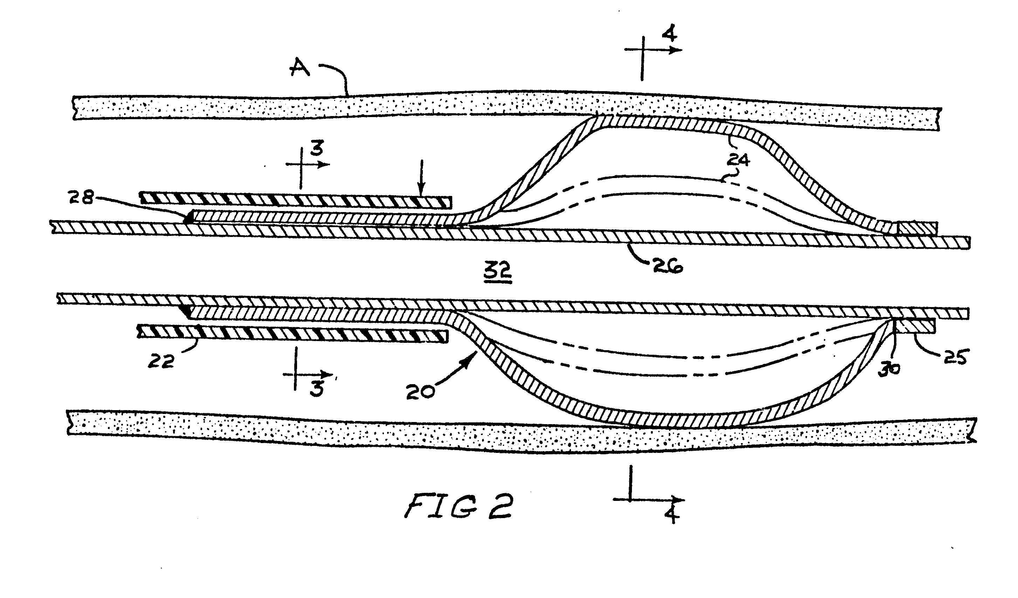

FIG. 2 shows the distal end of the centering catheter 20 within the unobstructed artery A with the centering catheter 20 having its spokes 24 extended to engage the artery's interior surface. When the centering catheter 20 is first advanced through the guiding catheter 10, the spokes 24 and the inside tube 26 are located entirely within the outside tube 22 with their distal ends at the same point. The proximal portion of this tunneling catheter system is then used to pull the outside tube 22 back until the spokes 24 are uncovered. As the spokes 24 move out, a slide ring 25 slides back along the inside tube 26 until the spokes 24 securely engage the interior surface of the arterial wall. At that point, the inside tube 26 will be centered within the unoccluded artery A. This can be further verified by fluoroscopy prior to proceeding with the tunneling. The spokes 24 have an attachment 28 to the inside tube 26 at their proximal end and have an attachment 30 to the slide ring 25 at the spokes 24 distal end. The region of the inside tube 26 that is in contact with the spokes 24 could be a collection of wires with one wire for each spoke. This construction would provide improved flexibility for passing through curving coronary arteries.

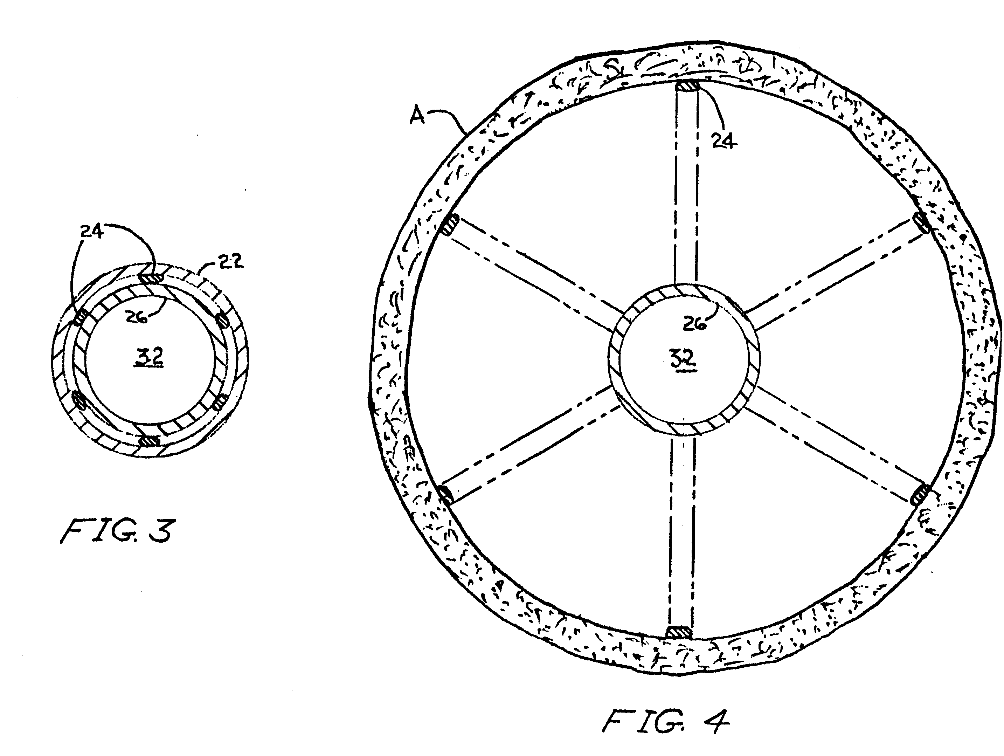

A lumen 32 within the inside tube 26 is used to pass the tunneling catheter 40. FIGS. 3 and 4 show cross-sections of the centering catheter 20. FIG. 3 shows the cross-section of the centering catheter 20 within the outside tube 22 as indicated by 3-3 of FIG. 2. FIG. 4 shows the cross-section of the centering catheter 20 at 4-4 of FIG. 2 showing the spokes 24 extended. Although 6 spokes are shown, 3 to 8 spokes could also be used for this centering purpose. This spoke design is such that each spoke 24 deploys with the same radial displacement for the same length of pull-back of the outside tube 22 relative to the inside tube 26. Hence, the inside tube 26 will be accurately centered within the artery A. However, the exact centering would be confirmed by fluoroscopy before tunneling would be attempted.

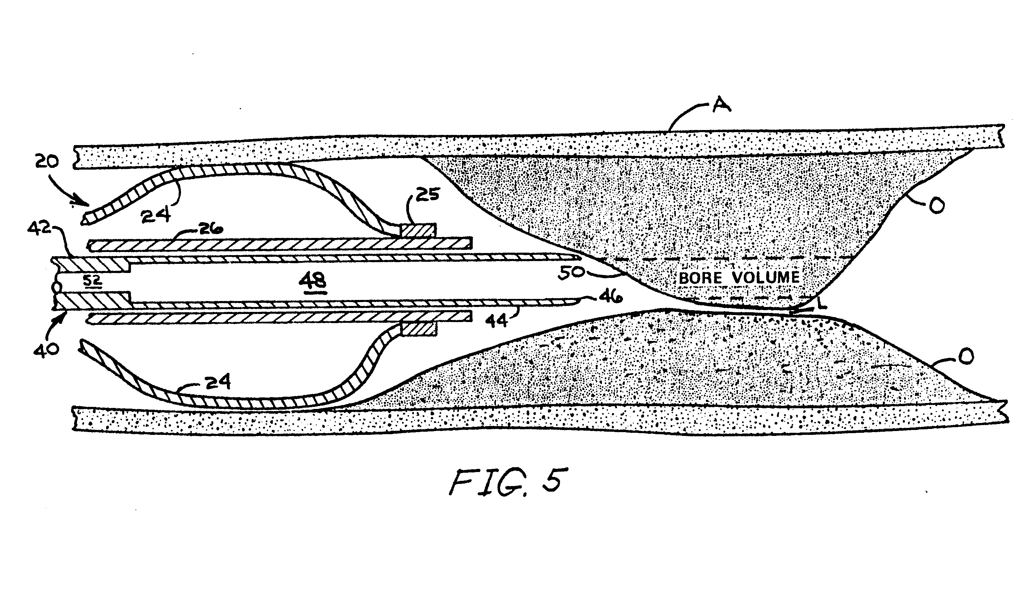

FIG. 5 shows the distal end of the centering catheter 20 located within an artery A just proximal to an occlusion O. A narrow and slightly eccentric lumen L is shown in the occlusion O. The spokes 24 have been extended to engage the interior surface of the artery A. A tunneling catheter 40 is shown protruding from the inside tube 26 of the centering catheter 20. The tunneling catheter 40 has a cutting edge 46, a distal section 44 containing a tunneling cavity 48 in fluid communication with an interior lumen 52 contained within the tunneling catheter tube 42. When the tunneling catheter 40 is advanced into the occlusion O, its cutting edge 46 will remove a bore volume 50 from the occlusion O. Because the spokes 24 allow blood flow through the restricted lumen L, which would not be the case if a balloon was used for centering, the doctor can work slowly and carefully to be sure that everything is properly positioned before the tunneling process is started. Furthermore, although the distal end 44 of the tunneling catheter 40 could possibly fully (or nearly fully occlude) blood flow while it is boring, its cutting action and retraction back into the inside tube 26 of centering catheter can be accomplished in a few seconds, thus, damaging ischemia of the distal tissue would be avoided. Of course, the technique described above would work equally well for an occlusion that had no opening whatsoever.

The distal end 44 of the tunneling catheter 40 can cut when its sharp edge 46 is advanced into a plaque or thrombotic occlusion. However, the cutting action can be improved by rotating the sharp edge 46 as it advances. This can be accomplished by rotation of a portion of the proximal end (outside the body) of the catheter system as will be described in the explanation of FIG. 14.

If a source of ultrasonic vibration is applied at the proximal end of the tunneling catheter 40, the sound energy will be transmitted to the cutting edge 46 thereby enhancing its ability to readily cut through even a calcified occlusion. This application of ultrasonic energy from the proximal end (outside the body) of the tunneling catheter 40 will be further explained herein with the aid of FIG. 14.

The centering catheter 20 would typically have its outside tube made from a plastic material such as teflon, nylon, or polyvinyl chloride. The outside diameter of the centering catheter 20 would vary according to the need for accessing different size arteries. The largest diameter might be 3 mm for clearing occlusions in the femoral artery in the leg; the smallest might be 1 mm outside diameter for entering a small coronary artery. The wall thickness of the outside tube 22 would typically be 0.2 mm for the largest catheters and 0.1 mm for the smallest. The inside tube 26 would be made to just pass inside the outside tube 22 and might be made of the same plastic materials. However, it may be desirable to make at least the distal end of the inside tube 26 out of 316 stainless steel with a wall thickness of approximately 0.05 mm. The spokes 24 would be made from 316 stainless steel with a thickness of approximately 0.1 mm and a width of 0.2 to 1.0 mm, depending on the diameter of the inside tube 26. The spokes 24 would be approximately 5 cm in total length. The slide ring 25 would have a thickness of approximately 0.1 mm, a length of approximately 1.0 mm, and a diameter that just fits around the outside surface of the inside tube 26. The distal end 44 of the tunneling catheter 40 would be made of 316 stainless steel for a length of about 2 cm. The tunneling catheter tube 42 may be made from a plastic material such as that used for the outside tube 22 of the centering catheter, or from a flexible piece of hollow 316 stainless steel wire, or from combinations of both materials.

The length of the guiding catheter 10, the centering catheter 20 and the tunneling catheter 40 would be approximately 120 cm.

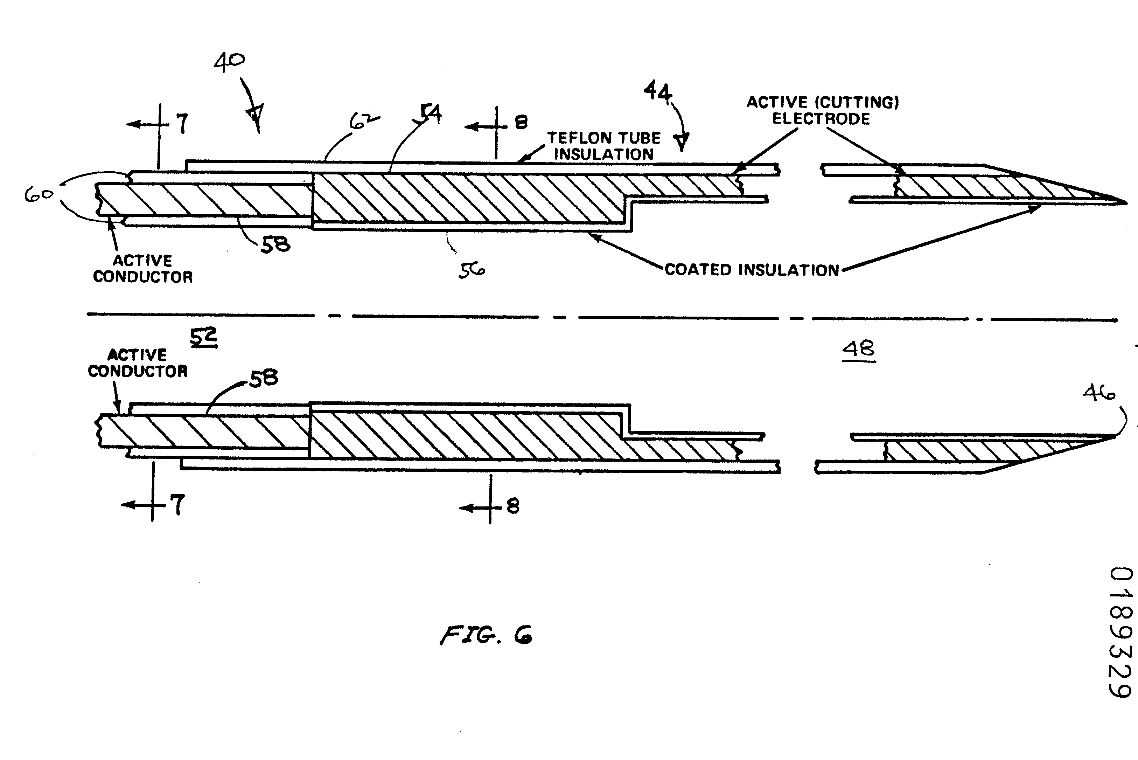

FIG. 6 shows the distal end 44 of a tunneling catheter 40 that can utilize an electrocautery cutting electric current at its sharp edge 46 to enhance the cutting action through an occlusion. An active, metallic cutting electrode 54 is conformal coated with electrical insulation 56 on its interior surface, and a thin, Teflon insulation 62 is placed around its exterior surface. The distal end is made into a sharp cutting edge 46. At the proximal end of the electrode 54 there are connected two electrical conductors 58 that are inside a cylindrically shaped electrical insulation 60. The proximal ends of the conductors 58 are connected to one terminal of an electrocautery current generating device (not shown) that is well known in the art. The other terminal of the electrocautery current generator is connected to a large area, indifferent (grounded) plate (not shown) that makes electrical contact with the patient's skin. Thus, the high concentration of electrocautery current will be at the sharp cutting edge 46 which provides effective cutting and cauterization as the sharp cutting edge 46 of the tunneling catheter 40 is advanced through the occlusion.

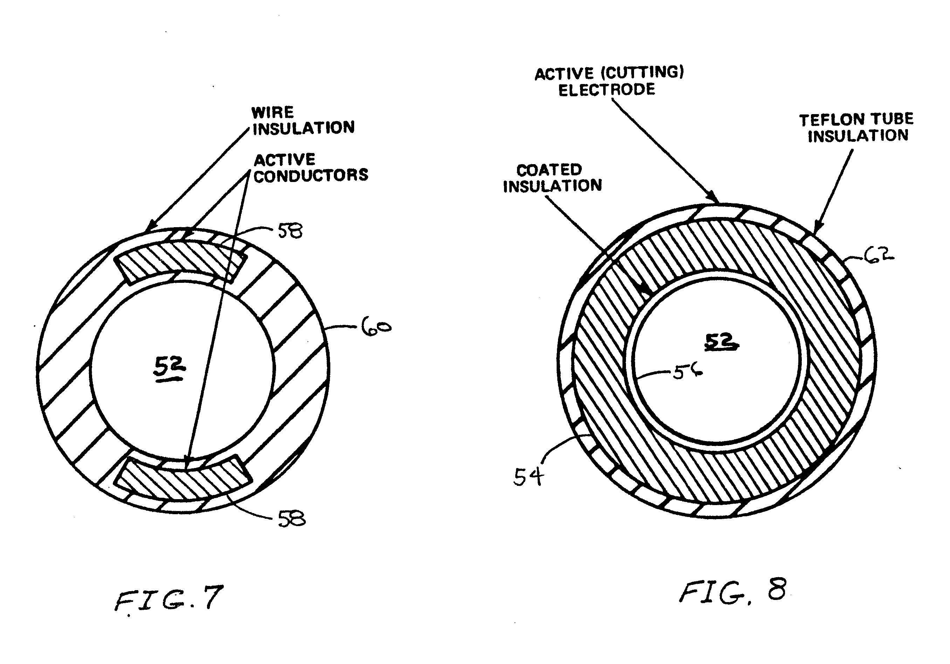

FIGS. 7 and 8 show cross sections of the electrocautery tunneling catheter 40 at 7-7 and 8-8 respectively of FIG. 6.

If the electrocautery tunneling catheter 40 of FIG. 6 is used in the femoral, carotid, renal or other peripheral arteries, there will be no possibility of inducing ventricular fibrillation as a result of the action of the electrocautery current on the heart. This is certainly true if the indifferent (grounding) plate electrode is placed on the skin in close proximity to the distal end 44 of the electrocautery tunneling catheter 40. For example, if tunneling through an occlusion in the carotid artery is desired, the indifferent plate electrode would be placed on the skin of the patient's neck.

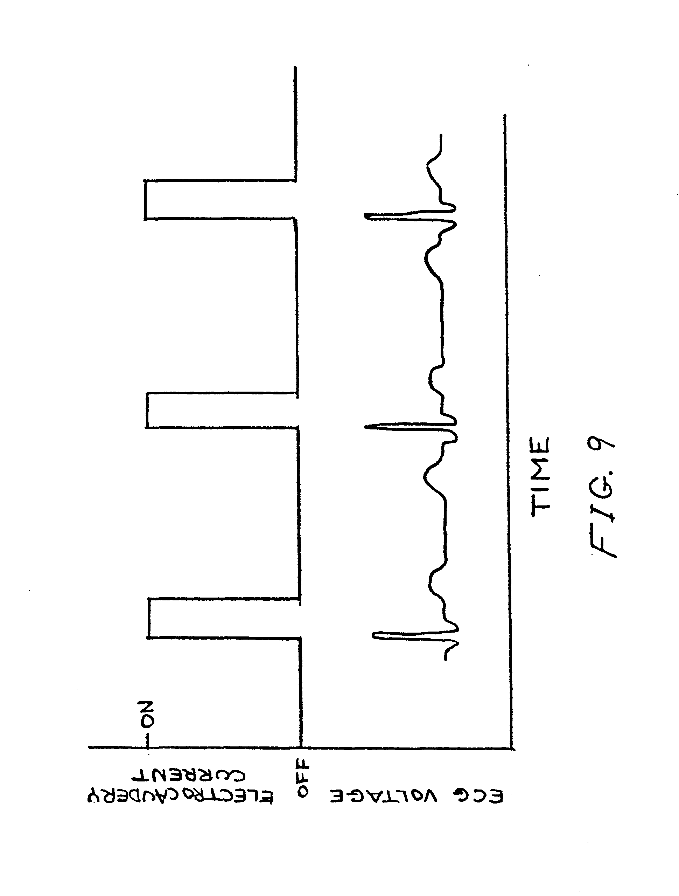

If however, it was desired to use an electrocautery catheter to tunnel through a coronary artery, it is possible that the electrocautery current could cause ventricular fibrillation. This can be avoided by timing the electrocautery current to be sychronously applied when the myocardium is in its refractory state, i.e., after the Q-wave and before the T-wave of the ECG. This timing of the electrocautery current relative to the ECG is illustrated in FIG. 9. The patients ECG would be monitored, and an electronic switching arrangement (that is well known in the electronic engineering art) would be used to apply the electrocautery current only when the heart is refractory to electrical inputs.

In FIG. 10 is shown the distal end 44 of the tunneling catheter 40 that can utilize a locally limited, bipolar electrocautery cutting electric current applied at the sharp edge 46 to enhance penetration of an occlusion. An inner conductor 64 of this tunneling catheter 40 is electrically isolated from the outer conductor 66 by an insulation 68. Furthermore, the distal end 44 is covered with a very thin insulation 70. When the proximal end of inner conductor 64 and outer conductor 66 are connected (through wire 72 and 74 respectively) to an electrocautery source (not shown) that is set for cutting, a high electric current density will occur at the sharp edge 46 allowing ready cutting through an occlusion. Both wires 72 and 74 are within a cylindrical insulator 76.

FIG. 11 is a cross section of this wire at 11-11 of FIG. 10.

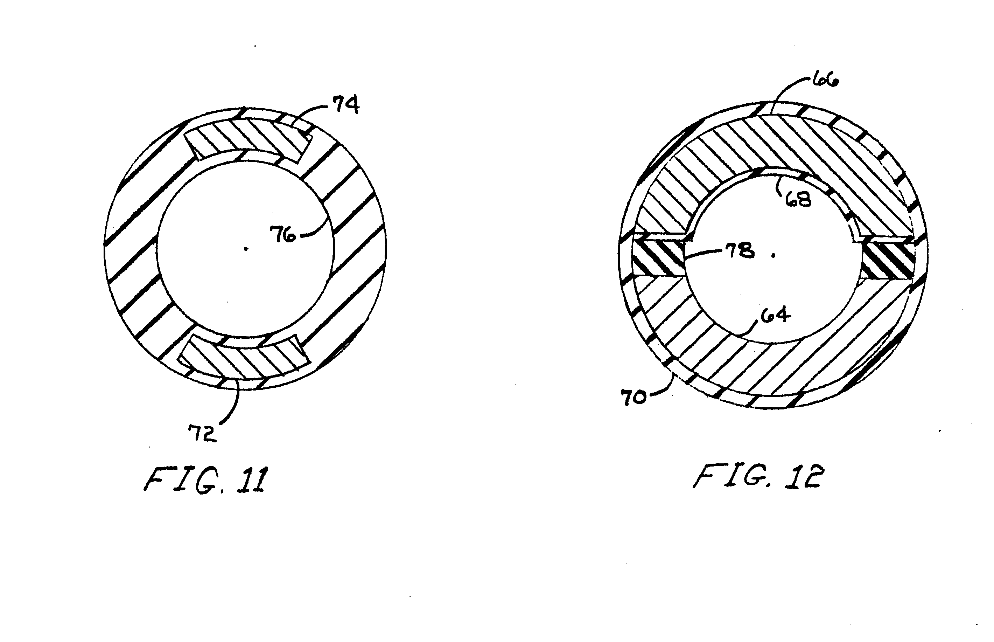

FIG. 12 is a cross section (at 12-12 of FIG. 10) showing how the inner electrode 64 and the outer electrode 66 are separated by an additional 78 that is not shown in FIG. 10.

This electrocautery construction minimizes the amount of stray electric current that could go through the heart, thus essentially eliminating the possibility that the heart would go into ventricular fibrillation or asystole as a result of the application of the electrocautery current. Of course this electrocautery tunneling catheter design could be used on peripheral as well as the coronary arteries.

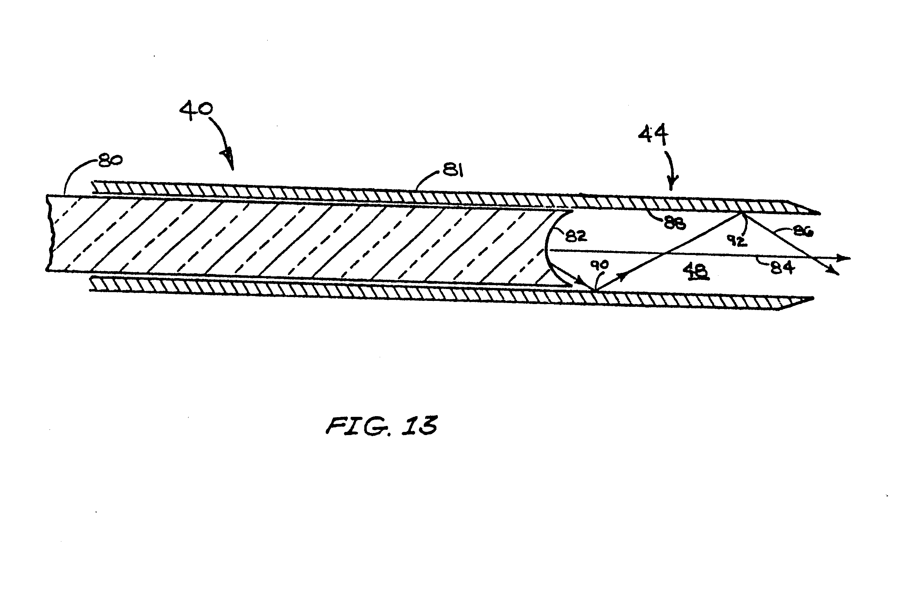

FIG. 13 shows the distal end 44 of a tunneling catheter 40 that utilizes a controlled laser light beam to vaporize plaque that enters the interior volume 48. Contained within an outside tube 81 is a fiber optic 80 whose distal end is a highly divergent lens 82 through which emanates a single center ray 84. One diverging ray 86 is also shown in FIG. 13. The lens is set back inside the cutting edge 46 a length equal to 4 to 20 diameters of the fiber optic 80. The laser light energy level is adjusted so that there is sufficient energy to vaporize the plaque only within, let us say 2 diameters in front of the diverging lens 82. The interior surface 85 of the distal end 44 is highly absorbing of laser light of the wavelength sent down the fiber optic 80. Thus, when the diverging rays (such as ray 86) strike the interior surface 88 (shown at points 90 and 92 in FIG. 13) the energy after each such reflection will be reduced by that absorption. In this way, the energy emanating from the end of the tunneling catheter 40 will be insufficient to vaporize plaque or, more to the point, will be insufficient to penetrate the arterial wall. Thus, unlike the invention of Choy, the laser light can vaporize the occlusion only when the stenotic material is forced into the confined and controlled space of the interior volume 48. Thus, inadvertent penetration of the arterial wall is precluded.

It should be noted here that a highly convergent (instead of divergent) lens at the distal end of the fiber optic 80 would operate in a similar manner. The interior surface 88 of the distal end 44 could be made absorbing of the laser light by coating it with a thin layer of black paint or by roughing up its surface. The source of laser light and its application at the catheter system's proximal end would be as described by Choy in US Patent 4,207,874 which techniques are well known in the art of laser light.

FIG. 14 illustrates the proximal end of the tunneling angioplasty catheter system. The proximal end of the guiding catheter 10 is shown to the right in FIG. 14. Not shown is the attachment of a side port of the guiding catheter 10 through which an angiographic dye material can be injected to the coronary arteries at various times during the procedure.

A stylet (not shown) is used to guide the guiding catheter 10 into a coronary artery such as the left coronary artery LCA (as shown in FIG. 1) as is well known in the medical art, and radio-opaque dye is used to verify the location of the occlusion and test all the fluoroscopy equipment. The stylet is then withdrawn from the interior lumen of the guiding catheter 10. The outside tube 22 of the centering catheter 20, attached by a cylindrical projection 80a to the centering catheter controller 80 is then advanced through the lumen of the guiding catheter 10 until its distal end is in contract with the proximal surface of the occlusion. A stylet (not shown) interior to the outside tube 22 can be used to guide the centering catheter 20 to its exact location within a coronary (or peripheral) artery. During this process, radio-opaque dye can be injected either into a side port (not shown) of the guiding catheter 10 or through the lumen of the outside tube 22 of the centering catheter 20.

The next step in this procedure (after the stylet is withdrawn from the outside tube 22) is to pass the inside tube 26 of the centering catheter 20 through the lumen of the outside tube 22 and advance the inside tube 26 until its distal end also makes contact with the occlusion. The inside tube 26 will have already been attached to the preassembled assembly consisting of the inside tube 26, the central controller 82, the outside tube 42 of tunneling catheter 40, the tunneling catheter controller 88 and the tunneling stroke length clamp ring 94. This assembly will have the centering catheter locking screw 86 unlocked and the tunneling release locking screw 92 and the tunneling clamp locking screw 98 both locked against the tunneling controller 88. This assembly will then be advanced into centering controller 80 until the front edge 82a of the central controller 82 is at the (0) position of the "PULL BACK" dial on the top surface 80b of the centering controller 80 (see FIG. 15). The centering controller 80 is then pushed back into the interior on the central controller 82 thus pulling back the outside tube 22 relative to the inside tube 26 and allowing the spokes 24 (of FIGS. 2, 3, 4, and 5) to expand against the inside surface of the artery as previously described herein. The "PULL BACK" stroke length will be determined by the diameter of the unoccluded arterial lumen. Thus sufficient radial deployment of the spokes 24 in a lumen diameter of 2.0 mm could be accomplished with (nominally) a 1.0 cm pull back and a 5.0 mm lumen diameter might require a 4.0 cm pull back. When the centering catheter 20 has its spokes 24 properly deployed, the centering catheter clamp screw 86 is advanced into the tapped hole 84 until it is locked onto the centering controller 80.

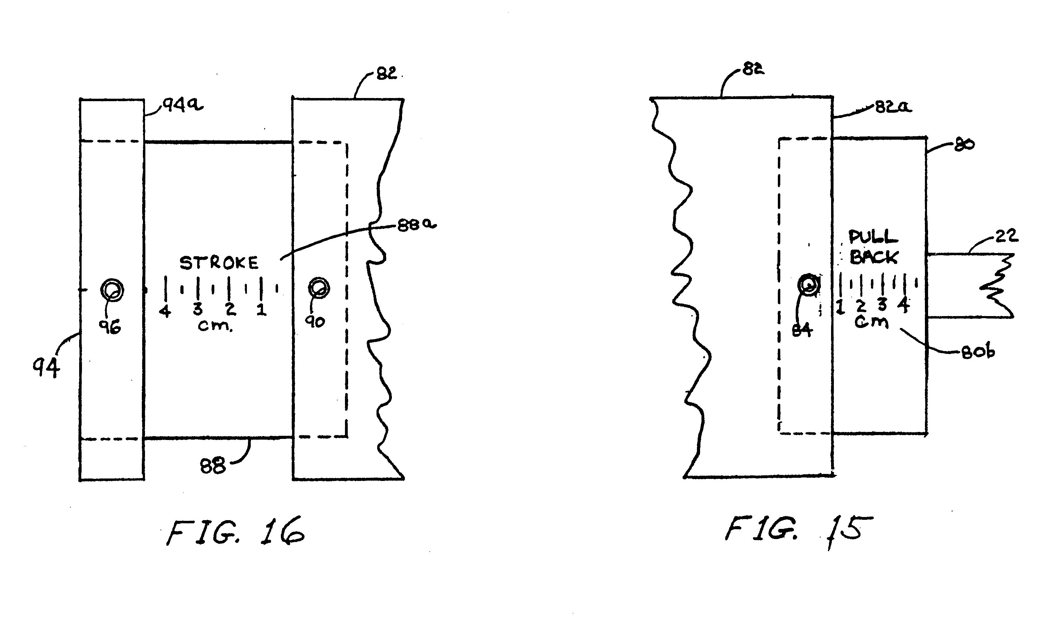

At this point radio-opaque dye is once more injected into the artery to confirm that the tunneling catheter's distal end 44 is indeed centered in the unoccluded arterial lumen, and to measure by fluoroscopy the length of the occlusion. When the measurement is made, let us say the occlusion is 2.2 cm long, the clamp ring locking screw 98 is loosened in its tapped hole 96, and the clamp ring is moved to the right (in FIGS. 14 and 16) until its front edge 94a is set at 2.2 cm on the "STROKE" dial which dial is shown on the top surface 88a of the tunneling controller 88 in FIG. 16.

The locking screw 98 is then advanced until it is locked against the tunneling controller 88. The locking screw 92 is then loosened and the tunneling controller 88 is pushed forward (while preferably rotating the cylindrically shaped tunneling controller) until the surface 82d and 94a are in contact. Hence, the cutting edge 46 of the tunneling catheter 40 will advance through the occlusion by that exact distance of 2.2 cm. This technique disallows the possibility that the sharp cutting edge 46 will be advanced beyond the occlusion and possibly penetrate the arterial wall with its concomitant unfortunate consequences.

The locking screw 92 is then released and the tunneling catheter 40 is then withdrawn to its "0" position and radio-opaque dye is then injected again into the guiding catheter 10 to see if an adequately long tunnel has been bored through the occlusion. If this has not been accomplished, a slightly increased stroke length would be set and the tunneling procedure would be repeated until a clearly cut through tunnel is confirmed by fluoroscopy.

If the blood flow through that tunnel is sufficient, the tunneling angioplasty catheter system is completely removed and the procedure is completed. If further dilation of the tunnel is desired, a larger diameter tunneling catheter 40 could be used and the tunneling procedure repeated, or a balloon angioplasty catheter which is well known in the medical art, could be positioned within the tunnel and then the balloon would be expanded to increase the size of the occluded lumen.

Returning now to FIG. 14, a suction source connected to the suction tubing 99 could be used to help hold the bore volume 50 (of FIG. 5) within the interior volume 48 of the tunneling catheter 40 as it is withdrawn from the body or to suck out any debris from the tunneling process. A rapid histologic examination of the bore sample might indicate if balloon angioplasty has a good chance of increasing the effective lumen, or would potentially endanger the patient by the breaking off of particles.

An ultrasonic energy source U (shown to the left in FIG. 9), could couple energy to the cutting edge 46 of the tunneling catheter 40 to enhance its ability to cut through calcified plaque. Also, ultrasonic energy could be applied through the body with its main energy focused at the cutting site. This technique of applying an ultrasonic vibration to the occlusion instead of to the catheter would also be effective in enhancing the cutting action of the catheter.

Various other modifications, adaptations and alternative designs are of course possible in light of the above techniques. Therefore, it should be understood at this time that within the scope of the appended claims, the invention may be practiced otherwise than as specifically described herein.

What is claimed is:

1.  A tunneling catheter system for percutaneous transluminal angioplasty, comprising:

    a centering means having an inner guide lumen and adapted to be advanced through a patient's arterial system to the vicinity of an arterial stenosis, said centering means for centering a distal portion of said inner guide lumen within said artery relative to the artery's inner surface; and

    a tunneling means having distal and proximal ends and adapted to slide within said inner guide lumen of said centering means, for tunneling through said arterial stenosis.

2.  The apparatus of claim 1, further comprising means for permitting the flow of blood through said centering means.

3.  The apparatus of claim 1, wherein said centering means further comprises a plurality of deployable radially extendable members, each member extendable an essentially equal radial distance into engagement with said artery's inner surface, deployment of said radially extendable members centering a distal portion of said inner guide lumen within said artery.

4.  The apparatus of claim 3, wherein said radially extendable members are spokes adapted to permit blood flow around said centering means.

5.  The apparatus of claim 1, wherein said distal end of said tunneling means includes a cutting means for tunneling a bore through said arterial stenosis.

6.    The apparatus of claim 5, wherein said tunneling means further comprises an ultrasonic means, operably coupled to said cutting means, for ultrasonically vibrating said cutting means thereby enhancing penetration of said cutting means into said arterial stenosis.

7.    The apparatus of claim 5, wherein said tunneling catheter further comprises an electrocautery means, operably coupled to said cutting means, for applying an electrical cautery current to said cutting means, thereby enhancing penetration of said cutting means into said arterial stenosis.

8.    The apparatus of claim 5, wherein said tunneling means further comprises an interior lumen extending from said proximal end of said tunneling means into fluid communication with said hollow tunneling cavity.

9.    The apparatus of claim 8, further comprising a suction means, located external to said patient, and operably coupled to said proximal end of said tunneling means, for applying suction through said interior lumen into said hollow tunneling cavity, thereby pulling plaque cut by said cutting means into said hollow tunneling cavity.

10.   The apparatus of claim 1, wherein said tunneling means is a tunneling catheter.

11.   The apparatus of claim 3, wherein said centering means is operably coupled to a control means, located external to said patient, for controlling the deployment and retraction of said radially extendable members.

12. The apparatus of claim 6, wherein said tunneling means is operably coupled to a control means for controlling movement of said cutting means.

13. The apparatus of claim 10, wherein said centering means comprises:

an outer tube having distal and proximal ends and having a hollow elongated bore;

an inner tube located within said bore of said outer tube and having distal and proximal ends and adapted to slide longitudinally within said bore, said inner tube also having said inner guide lumen for guiding said tunneling catheter; and

a deployment means associated with said inner and said outer tubes for deploying said radially extendable members when said distal end of said outer tube is displaced proximally from said distal end of said inner tube and for retracting said radially extendable members when displacement between said distal ends of said inner and outer tubes is decreased.

14. The apparatus of claim 1, wherein said tunneling means comprises a tunneling catheter having distal and proximal ends and adapted to slide within said inner guide lumen of said centering means, said tunneling catheter further comprising, a tube with distal and proximal ends and having an elongated interior lumen, said distal end of said tube adapted to be advanced to the vicinity of said arterial stenosis through said inner guide lumen of said centering means, the proximal end of said tube extending external to said patient, a distal section of said tube having a tunneling cavity, said tunneling cavity being in fluid communication with said proximal end of said tube through said interior lumen, said tunneling catheter further including a cutting edge located at said distal edge of said tube and forming a mouth of said tunneling cavity.

15. The apparatus of claim 14, wherein said proximal end of said tube is operably coupled to a suction means located external to said patient, for applying suction to said interior lumen and said tunneling cavity for pulling said cylindrical bore of plaque into said tunneling cavity.

16. The apparatus of claim 14, wherein said proximal end of said tube is operably coupled to a fluid injection means, located external to said patient, for injecting through said interior lumen and through said tunneling cavity radio-opaque dye into the vicinity of said arterial stenosis.

17. The apparatus of claim 14, wherein said proximal end of said tube is operably coupled to a control means, located external to said patient, for controlling advancement of said cutting edge longitudinally relative to said centering mean's distal end, thereby controlling cutting action of said cutting edge through said plaque.

18. A tunneling catheter for percutaneous transluminal angioplasty, comprising:

a catheter with distal and proximal ends and having an elongated interior lumen, said distal end of said catheter adapted to be advanced to the vicinity of a patient's arterial stenosis, said proximal end of said catheter extending external to said patient, a distal section of said catheter having a tunneling cavity, said tunneling cavity being in fluid communication with said proximal end of said catheter through said interior lumen; and

a cutting edge located at said distal edge of said catheter and forming a mouth of said tunneling cavity.

19. The apparatus of claim 18, wherein said cutting edge is cylindrical for cutting a cylindrical bore of plaque from said arterial stenosis.

20. The apparatus of claim 19, wherein said proximal end of said catheter is operably coupled to a suction means located external to said patient, for applying suction to said interior lumen and said tunneling cavity for pulling said cylindrical bore of plaque into said tunneling cavity.

21. A tunneling electrocautery catheter for percutaneous transluminal angioplasty, comprising:

a tunneling catheter with distal and proximal ends and having an elongated interior lumen, said distal end of said tunneling catheter adapted to be advanced to the vicinity of a patient's arterial stenosis, said proximal end of said tunneling catheter extending external to said patient;

an active cylindrical electrode attached to said distal end of said tunneling catheter, said active cylindrical electrode's distal end tapered to a sharp cutting edge, said active cylindrical electrode having a tunneling cavity extending proximally to said sharp

0189329

- 27 -

cutting edge, said tunneling cavity being in fluid communication with said interior lumen of said tunneling catheter; and

a means for conducting electrical energy to said active cylindrical electrode.

22. A procedure for percutaneous transluminal angioplasty comprising the steps of:

advancing a guiding catheter having a hollow center through a patient's arterial system to the vicinity of a stenosis in an artery;

sliding a centering catheter, having distal and proximal ends and having an inner guide lumen, through said hollow center of said guiding catheter to a position in contact with a proximal surface of said arterial stenosis, said distal end of said centering catheter having a centering means for centering said centering catheter relative to said artery's inner surface;

centering said centering catheter by said centering means;

passing a tunneling catheter having distal and proximal ends through said inner guide lumen of said centering catheter until said distal end of said tunneling catheter is aligned with said distal end of said centering means; and

cutting a bore of plaque from said arterial stenosis by advancing said tunneling catheter a certain stroke distance, a distal portion of said tunneling catheter having a tunneling cavity and including a cutting means for cutting through said arterial stenosis and for retaining said bore of plaque in said tunneling cavity.

23. The procedure of claim 22, further including, after said cutting step, the step of:

withdrawing said tunneling catheter from said centering catheter with said bore of plaque retained in said tunneling cavity.

24. The procedure of claim 23, further including the steps of:

inserting a balloon catheter through said inner guide lumen of said centering catheter into a bore hole cut by said tunneling catheter; and,

inflating said distal end of said balloon catheter to further enlarge said bore hole.

25. The procedure of claim 22, wherein said centering means comprises a plurality of deployable radially extendable members, for engagement with said artery's inner surface.

26. The procedure of claim 25, wherein said centering catheter further includes a control fixture means, located external to said patient, for controlling radial deployment of said radially extendable members, said procedure further including, subsequent to said sliding step, the step of

deploying each of said radially extendable members an essentially equal radial displacement into engagement with said inner surface of said artery by adjusting said control fixture means and thereby centering a distal portion of said centering catheter within said artery.

FIG. 1

FIG 2

FIG. 3

FIG. 4

0189329

FIG. 5

FIG. 6

WIRE
INSULATION

ACTIVE
CONDUCTORS

58

60

**52**

58

*FIG. 7*

ACTIVE (CUTTING)
ELECTRODE

TEFLON TUBE
INSULATION

COATED
INSULATION

62

**52**

56

54

*FIG. 8*

6/12

0189329

FIG. 9

FIG. 10

66

68

78

94

70

FIG. 12

74

76

72

FIG. 11

FIG. 13

FIG. 14

0189329

94a

82

88a

STROKE

4 3 2 1
cm.

96

90

94

88

FIG. 16

82

82a

88

PULL
BACK

1 2 3 4
cm

84

22

80b

FIG. 15